# FASCICULE DE BREVET EUROPEEN

(11) **EP 3 810 157 B1**
(45) Date de publication et mention de la délivrance du brevet: **02.11.2022**
(21) Numéro de dépôt: 19734724.8
(22) Date de dépôt: 20.06.2019
(51) Int. Cl.: A61K 35/15, C12N 5/0786, A61P 35/00

(54) **SURNAGEANT DE COCULTURE ENTRE MACROPHAGES ET LEUCOCYTES IRRADIÉS UTILISÉ POUR CONTROLER UNE PROGRESSION TUMORALE OU POUR RESTAURER UNE IMMUNITE ANTI-TUMORALE**
KULTURÜBERSTAND VON CO-KULTUR VON MAKROPHAGEN UND BESTRAHLTE LEUKOZYTEN ZUR ZUR VERRINGERUNG DER TUMORPROGRESSION ODER ZUR WIEDERHERSTELLUNG VON TUMORIMMUNITÄT
SUPERNATANT OF COCULTURE OF MACROPHAGES AND IRRADIATED LEUCOCYTES FOR USE FOR CONTROLLING TUMOR PROGRESSION OR RESTORE ANTI-TUMORAL IMMUNITY

(30) Priorité: 21.06.2018 FR 1800636
(43) Date de publication de la demande: 28.04.2021
(73) Titulaire: Med' Inn' Pharma, 25170 Placey (FR)
(72) Inventeur: PERRUCHE, Sylvain, 25170 Placey (FR); BONNEFOY, Francis, 25870 Les Auxons (FR); COUTURIER, Mélanie, 70230 Vy-Les-Filain (FR)
(74) Mandataire: Cabinet Netter
(86) Numéro de dépôt international: PCT/EP2019/066406
(87) Numéro de publication internationale: WO 2019/243544

(56) Documents cités:
- EP-A1- 2 941 257
- WO-A2-01/28573
- SHIBATA T ET AL: "Apoptotic neutrophils and nitric oxide regulate cytokine production by IFN-@c-stimulated macrophages", CYTOKINE, ACADEMIC PRESS LTD, PHILADELPHIA, PA, US, vol. 53, no. 2, 1 février 2011 (2011-02-01), pages 191-195, XP027590037, ISSN: 1043-4666 [extrait le 2011-01-10]
- ZHANG QI ET AL: "Resolution of Cancer-Promoting Inflammation: A New Approach for Anticancer Therapy.", FRONTIERS IN IMMUNOLOGY 2017, vol. 8, 2017, page 71, XP002789002, ISSN: 1664-3224
- Bonnefoy Francis: "PRO-Resolving Mediators Issued from Apoptotic CELL Efferocytosis (SUPERMAPO) Modulate Antigen Presenting CELL Properties Toward a Tolerogenic Profile: Efficacy in the Treatment of Collagen- Induced Arthritis - ACR Meeting Abstracts", 2015 ACR/ARHP Annual Meeting, 29 September 2015 (2015-09-29), XP055918197, Retrieved from the Internet: URL:https://acrabstracts.org/abstract/pro- resolving-mediators-issued-from-apoptotic- cell-efferocytosis-supermapo-modulate-anti gen-presenting-cell-properties-toward-a-to lerogenic-profile-efficacy-in-the-treatmen t-of-collagen-induced-ar/>

## Description

La présente invention concerne une méthode de résolution de l'inflammation pro-tumorale à l'aide d'une préparation pharmaceutique.

La réaction inflammatoire est un processus biologique impliqué dans des réactions naturelles de défense de l'organisme mais aussi associé à une multitude de réactions pathologiques.

L'inflammation évoque en particulier des maladies inflammatoires telles que les maladies inflammatoires de l'intestin comme la maladie de Crohn et la poly arthrite rhumatoïde, mais elle est également impliquée dans la pathogenèse de l'obésité, du diabète et des troubles neurologiques tels que la dépression. Les facteurs pro-inflammatoires comme les cytokines pro-inflammatoires participent et soutiennent l'inflammation dans l'obésité, la tumorigenèse et au cours de la progression du cancer. En effet, l'inflammation chronique joue un rôle important dans la promotion de l'échappement de la tumeur au système immunitaire et au maintien du développement de la tumeur. L'inflammation reste un facteur de risque aigu pour le cancer.

L'inflammation chronique peut être apparentée à une résolution partielle ou non aboutie de la réaction inflammatoire, qui peut être favorisée par des facteurs génétiques, environnementaux ou infectieux. L'inflammation chronique est particulièrement impliquée dans la progression des cancers induits par infections virales, comme l'inflammation associée à l'infection au papilloma virus (VPH) qui augmente le risque de cancer du cou, à celle associée au virus de l'hépatite B et C et l'apparition d'hépatocarcinome ou encore avec Helicobacter pylori et le développement d'adénocarcinome ou de lymphome. L'inflammation chronique dans les maladies auto-immunes telles que la maladie de Crohn ou la colite ulcéreuse est aussi associée à un risque plus élevé de développer un cancer colorectal. Enfin, l'inflammation induite par une exposition aux irritants ou dans l'obésité favorise également une activité pro-tumorale.

Ainsi, lors de l'agression de l'organisme provoquant une inflammation, il est important de provoquer la résolution de l'inflammation ; si la résolution de l'inflammation n'est pas complète, une inflammation chronique peut s'installer, débouchant sur les pathologies précitées.

Si les médiateurs inflammatoires sont essentiels à la surveillance anti-tumorale, notamment au cours de la phase d'immuno-édition cancéreuse permettant la mise en place de réponses immunitaires anti-tumorales adaptées, ces derniers peuvent jouer un rôle pro-tumoral, et en particulier le TNF (tumor necrosis factor). En effet, le microenvironnement inflammatoire tumoral peut favoriser la croissance tumorale, l'angiogenèse, l'invasion et la formation de métastases. Le développement tumoral est une conséquence de l'invasion des cellules tumorales en raison d'une prolifération accrue des cellules cancéreuses, de leur résistance à la mort par apoptose et à une angiogenèse dans le microenvironnement tumoral.

Par exemple, le TNF pourra favoriser l'attraction des lymphocytes T régulateurs (Tregs) dans la tumeur ainsi que celle des cellules myéloïdes suppressives (MDSCs), affaiblissant davantage la réponse anti-tumorale. Paradoxalement, alors que les thérapies cytotoxiques induisant une mort immunogène des cellules tumorales ont permis de démontrer un rôle majeur du système immunitaire dans le choix des traitements anticancéreux conventionnels, ces mêmes approches sont maintenant décrites comme associant des effets secondaires pro-tumoraux à travers les cellules mortes tuées par ces thérapies qui stimuleraient la croissance tumorale.

Dans les maladies inflammatoires chroniques, la dérégulation de la résolution de l'inflammation reste également une piste thérapeutique de choix afin de réenclencher la résolution naturelle de l'inflammation, c'est-à-dire permettre l'arrêt de la réponse inflammatoire. Actuellement, les approches thérapeutiques ciblées neutralisant ou détruisant les facteurs inflammatoires ont montré leurs limites. De plus il semble illusoire de pourvoir contrôler une pathologie complexe comme la maladie de Crohn en neutralisant uniquement un facteur inflammatoire, qui n'est qu'une conséquence de la pathologie. En revanche, résoudre l'inflammation en rétablissant une résolution efficace pourrait permettre de traiter le cœur de la réaction inflammatoire.

Ainsi encourager la résolution de l'inflammation et améliorer l'élimination des cellules tumorales tuées par les traitements anticancéreux deviennent un enjeu majeur dans la prise en charge du cancer. De la même manière cibler la résolution de l'inflammation pourrait permettre un contrôle à la racine de la pathologie inflammatoire.

L'invention concerne notamment des méthodes de prévention du cancer utilisant l'administration d'un résolutome, c'est-à-dire un ensemble de facteurs pro-résolutifs, capables de déclencher la résolution de l'inflammation, en inhibant l'échappement de la tumeur à la surveillance immunitaire et son développement, et en rétablissant une immunité anti-tumorale efficace.

Le brevet EP 2 941 257 décrit une préparation pharmaceutique en vue de son utilisation dans la prévention ou le traitement d'une réponse immunitaire pathologique ; cette préparation pharmaceutique comprend un surnageant pouvant être obtenu à partir de la coculture de phagocytes avec des cellules apoptotiques. Dans ce brevet, la préparation pharmaceutique est utilisée dans le traitement d'une pathologie telle qu'une maladie auto-immune, par exemple de la polyarthrite rhumatoïde (PR) ou des maladies inflammatoires chroniques de l'intestin (MICI). Cette préparation pharmaceutique est également utilisée pour traiter la maladie du greffon contre l'hôte (MGCH).

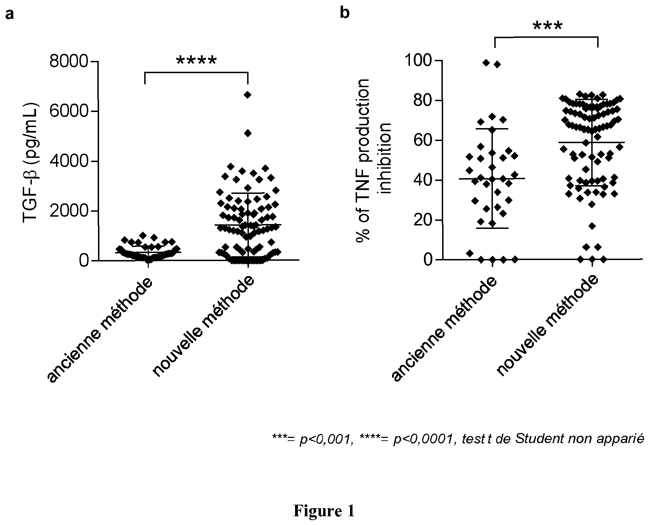

La présente invention a une cible différente, à savoir une méthode de résolution de l'inflammation pro-tumorale à l'aide d'un surnageant obtenu par une coculture de macrophages et de leucocytes irradiés ; ce surnageant comporte de façon inattendue de nombreux avantages par rapport au surnageant décrit dans le brevet EP 2 941 257. En particulier, il offre une quantité en facteurs pro-résolutifs plus importante que dans le cas du surnageant du brevet précité (Figure la) ; il présente également une supériorité biologique (Figure 1b).

L'utilisation de la préparation pharmaceutique selon l'invention se fait au moment d'une résolution défaillante afin de rétablir une résolution efficace et permettre un arrêt de la réaction inflammatoire. Les buts de l'invention sont donc en particulier de résoudre une inflammation chronique installée et résoudre une inflammation favorisant la croissance tumorale à l'aide de la préparation pharmaceutique précitée.

La présente invention est d'abord basée sur la démonstration que de cibler la résolution de l'inflammation pro-tumorale permet de restaurer la réponse immunitaire anti-tumorale et d'arrêter la réaction inflammatoire chronique.

Il est rappelé à ce stade que l'inflammation joue un rôle bénéfique au début de l'installation d'une tumeur puis négatif par la suite favorisant l'échappement, ce que tend à contrôler la présente invention.

Une méthode selon la présente invention consiste à utiliser un surnageant obtenu en générant des macrophages dérivés de monocytes à partir de leucocytes du sang, de les stocker avec des leucocytes du sang préalablement irradiés permettant ainsi leur coculture et de récolter les facteurs contenus dans le surnageant produits par les cellules.

Le surnageant constituant la préparation pharmaceutique selon l'invention destinée à obtenir la résolution de l'inflammation, sera dénommé « Resolvix » dans la suite de la présente description.

L'injection de ces facteurs permet de résoudre la réaction inflammatoire en cours dans plusieurs modèles expérimentaux (arthrite, inflammation des intestins - IBD, colite, encéphalomyélite auto-immune expérimentale), mais aussi de favoriser la réparation des tissus. De façon intéressante, cette approche permet aussi la restauration de l'immunité anti-cancéreuse et ainsi de favoriser la régression tumorale.

A titre d'exemple non limitatif, une méthode de production du surnageant selon l'invention (Resolvix) va maintenant être décrite.

Les leucocytes sont isolés à J0 par gradient de densité à partir de sang total, lavés, re-suspendus dans un milieu de culture défini, classiquement utilisé par l'homme du métier dans le présent domaine technique, à base de sels, d'acides aminés et de vitamines, comme par exemple celui connu sous le nom RPMI 1640, puis répartis à 60% dans une première poche et à 40% dans une seconde. La première poche reçoit alors un facteur de différenciation, pouvant être du M-CSF (Macrophages Colony-Stimulating Factor), et est conservée dans des conditions préservant la viabilité cellulaire, par exemple à 37°C, 5% de CO₂, pendant plusieurs jours, préférentiellement 3, puis reçoit à nouveau du milieu de culture défini frais précité. Cette première poche est alors laissée en l'état, aboutissant à la production de macrophages après plusieurs jours de conservation, préférentiellement 4 jours, dans des conditions préservant la viabilité cellulaire, par exemple à 37°C, 5% de CO₂. La seconde poche est quant à elle conservée à basse température, préférentiellement à une température inférieure à +10°C, et encore plus préférentiellement inférieure à +5°C. Au 7^{ème} jour, la seconde poche est irradiée, par exemple à l'aide de rayons X, puis la première et la seconde poche sont débarrassées de leur milieu. La seconde poche est alors reprise dans un milieu de sécrétion, à base notamment de sels, d'acides aminés et de vitamines, connu de l'homme du métier, comme par exemple celui connu sous le nom MEM, et transférée dans la première pour un stockage dans des conditions préservant la viabilité cellulaire, par exemple à 37°C, 5% de CO₂. Lors de ce stockage la coculture a lieu aboutissant à la production d'un surnageant conditionné, ce qui peut prendre plusieurs jours, préférentiellement 2. Dans la présente description, on entend par « surnageant conditionné » un surnageant contenant des facteurs dérivés des cellules. A l'issue de cette période de coculture, le surnageant est collecté dans une nouvelle poche, filtré puis conservé à très basse température, préférentiellement aux environs de -80°C. Ce surnageant est alors avantageusement mélangé avec d'autres surnageants produits, selon la méthode précédemment décrite, à partir de couches leuco-plaquettaires issues d'autres donneurs de sang, puis ce mélange est réparti en doses unitaires, lyophilisées ou non, puis stockées.

De façon plus précise, des modèles de croissance tumorale in vivo ont été utilisés. Les résultats obtenus sont repris dans les figures suivantes :
- **Figure 1** : comparaison biologique et d'activité de la nouvelle méthode de production aboutissant au surnageant dénommé Resolvix. La nouvelle méthode de production permet l'obtention d'une quantité supérieure en facteurs pro-résolutifs comme la cytokine anti-inflammatoire TGF-beta, quantifiée par test ELISA (a), et une supériorité biologique (b) évaluée par test biologique d'inhibition de production de TNF dans des monocytes stimulés par phytohaemagglutinin. Données issues de 35 échantillons ancienne méthode et 89 nouvelle méthode, exprimées en % d'inhibition de production de TNF, représentés avec la moyenne +/l'erreur standard sur la moyenne. ^{∗∗∗}= p<0,001, ^{∗∗∗∗}= p<0,0001, test t de Student non apparié. Dans cette Figure, l'ancienne méthode se rapporte à la méthode aboutissant au surnageant décrit dans le brevet EP 2 941 257 précité.

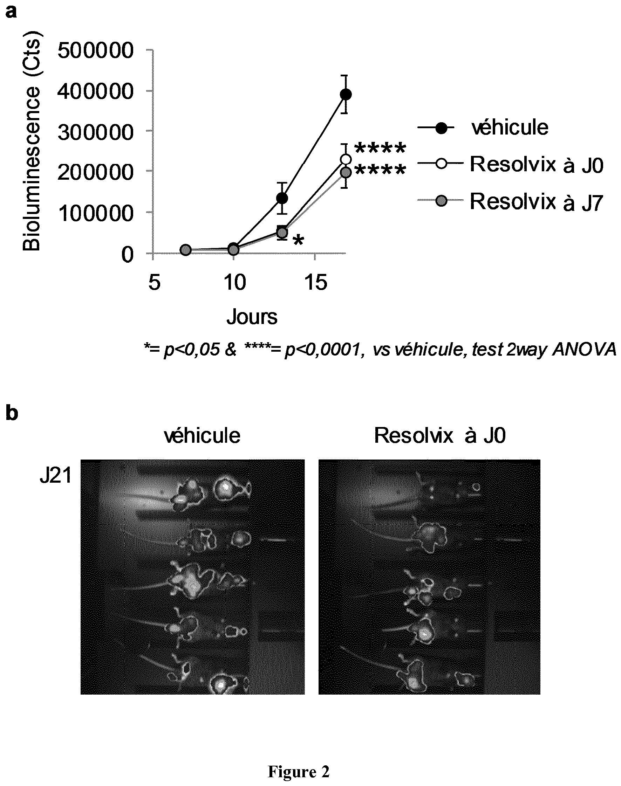

- **Figure 2** : suivi de la croissance tumorale. La bioluminescence émise par la lignée tumorale EL4-Luc+ (après injection de luciférase) a été quantifiée chez des souris C57Bl/6 leucémiques, traitées ou non par Resolvix à J0 ou J7 après injection des cellules leucémiques (a). Données issues d'une expérience représentative de deux avec 5 animaux par groupe, exprimées en moyenne de groupe +/- erreur standard sur la moyenne. ^{∗}= p<0,05, ^{∗∗∗∗}= p<0,0001, vs véhicule, test 2way ANOVA. Des images représentatives de bioluminescence de 5 souris non traitées (véhicule) et de 5 souris ayant reçu Resolvix à J0, acquises 21 jours après injection des cellules tumorales, sont montrées (b). L'intensité de la luminescence émise (du bleu foncé au jaune) est proportionnellement associée au nombre de cellules tumorales ayant proliférées.

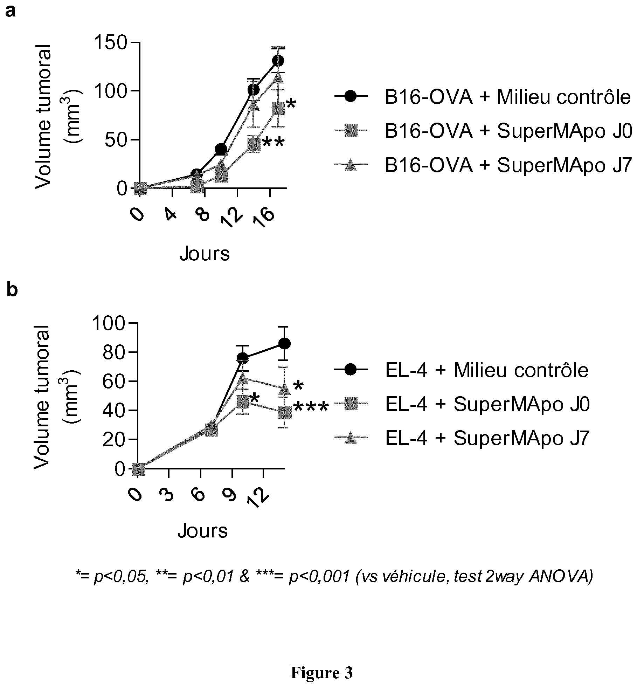

- **Figure 3** **:** évolution des tumeurs après traitement par Resolvix. Le volume des tumeurs (en mm3) a été évalué dès l'injection de la lignée tumorale B16-OVA (a) ou de la lignée tumorale EL-4 (b) en sous-cutané dans le flanc abdominal droit de souris C57Bl/6, au cours du temps. Resolvix a été injecté soit le jour de l'injection des cellules cancéreuses (J0), soit 7 jours après (J7). Données issues de 2 expériences (a et b) représentatives de 6 avec 5 animaux par groupe, exprimées en moyenne +/- erreur standard sur la moyenne. ^{∗}= p<0,05, ^{∗∗}= p<0,01 & ^{∗∗∗}= p<0,001 vs véhicule, test 2way ANOVA.

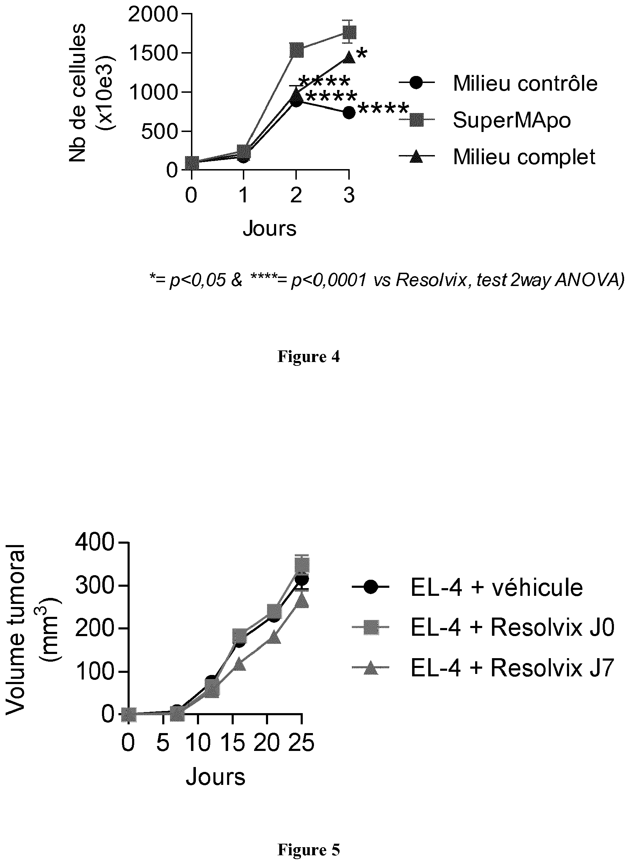

- **Figure 4** : évolution de la prolifération cellulaire in vitro de la lignée cellulaire cancéreuse EL-4 en présence de Resolvix. La prolifération cellulaire a été évaluée par un comptage cellulaire tous les jours pendant 3 jours. Les données sont issues d'une expérience représentative de deux, exprimées en moyenne de triplicats +/- erreur standard sur la moyenne. ^{∗}= p<0,05, ^{∗∗∗∗}= p<0,0001 vs Resolvix, test 2way ANOVA.
- **Figure 5** : traitement Resolvix de tumeurs solides chez des souris immunodéficientes. Le volume (en mm3), des tumeurs induites à partir de la lignée cancéreuse EL-4 en sous-cutané dans le flanc abdominal droit de souris immunodéficientes RAG-y/c, a été déterminé à différents temps. Resolvix a été injecté soit le jour de l'injection des cellules cancéreuses (J0) ou 7 jours après (J7). Les données sont issues d'une expérience avec 5 animaux par groupe, exprimées en moyenne +/- erreur standard sur la moyenne.

Ces données démontrent de façon intéressante, que l'injection de Resolvix chez des souris porteuses de la leucémie à lymphome T (lignée tumorale EL4-luciférase+ permettant le suivi de la croissance tumorale par bio-imageur) aboutit à une régression de la croissance tumorale (Figure 1).

Ces mêmes observations ont été faites à l'aide d'un second modèle, avec des tumeurs solides (lignées tumorales EL-4 ou B16-OVA injectées de façon sous-cutanée), dans lequel l'injection de Resolvix permet la réduction de la taille des tumeurs (Figure 2).

Ces résultats suggèrent que le traitement Resolvix pourrait influencer directement la croissance de la lignée tumorale in vivo, par un effet cytotoxique direct. Afin d'évaluer cette possibilité, des expériences supplémentaires in vitro ont été réalisées et ont montré que la culture des cellules tumorales EL-4 en présence de Resolvix favorise au contraire la croissance de la lignée (Figure 3). Ces données montrent que l'inhibition de la croissance tumorale observée in vivo après traitement par Resolvix n'est pas liée à un effet cytotoxique direct du médicament sur les cellules tumorales.

De plus, l'effet du traitement n'étant pas direct sur les cellules tumorales, ceci suggère la restauration d'une immunité anti-tumorale par le traitement. En effet, en absence de système immunitaire, c'est-à-dire chez des souris immunodéficientes C57B1/6 RAG-y/c porteuses de tumeurs, le traitement Resolvix n'a aucune incidence sur la croissance tumorale in vivo (Figure 4).

L'injection de Resolvix dans les modèles tumoraux est réalisée au moment de la greffe des cellules tumorales (J0) ou 7 jours après (J7). En contrôlant ainsi l'inflammation à ces différents temps, il est possible de contrôler la croissance tumorale et rétablir une immunité anti-tumorale.

Ainsi, il ressort de ce qui précède, que l'utilisation du Resolvix selon la présente invention permet de restaurer l'immunité anti-tumorale et/ou de stopper la progression tumorale. Plus généralement, la préparation pharmaceutique selon l'invention pourra être utilisée en vue de cibler, contrôler, inhiber, résoudre une inflammation associée au cancer, permettant, ainsi que cela vient d'être mentionné, de restaurer l'immunité anti-tumorale et/ou de stopper la progression tumorale.

Le surnageant selon l'invention trouvera notamment une application dans le traitement de cancers, seul ou en complément d'autres thérapies, en médecine humaine ou vétérinaire. Les cancers sont notamment ceux représentatifs des lymphomes, leucémies, sarcomes et carcinomes, et plus précisément des lymphomes à cellules T, des lymphomes à cellules B, des mélanomes et des carcinomes du colon.

## Revendications

1. Préparation pharmaceutique pour utilisation pour contrôler une progression tumorale ou pour restaurer une immunité anti-tumorale, comportant un surnageant obtenu à partir d'une coculture entre des macrophages et des leucocytes irradiés.

2. Préparation pharmaceutique selon la revendication 1, **caractérisée en ce que** lesdits macrophages sont des leucocytes.

3. Préparation pharmaceutique selon les revendications 1 et 2 **caractérisée en ce que** lesdits leucocytes sont isolés de la couche leuco-plaquettaire issue de sang total.

4. Préparation pharmaceutique selon la revendication 3, **caractérisée en ce qu'**une partie desdits leucocytes est lavée, re-suspendue dans un milieu de culture défini, puis placée dans une première poche recevant un facteur de différenciation et conservée dans des conditions préservant la viabilité cellulaire pendant plusieurs jours, préférentiellement 3, avant de recevoir à nouveau du milieu de culture défini frais, puis être laissée en l'état pendant à nouveau plusieurs jours, préférentiellement 4, aboutissant à la production de macrophages.

5. Préparation pharmaceutique selon la revendication 3, **caractérisée en ce qu'**une autre partie desdits leucocytes est placée dans une seconde poche conservée à basse température, préférentiellement inférieure à +10°C, et encore plus préférentiellement inférieure à +5°C.

6. Préparation pharmaceutique selon la revendication 5, **caractérisée en ce que** ladite seconde poche après avoir été débarrassée de son milieu irradiée, est débarrassée de son milieu, puis reprise dans un milieu de sécrétion dans des conditions préservant la viabilité cellulaire.

7. Préparation pharmaceutique selon la revendication 6, **caractérisée en ce que** ladite seconde poche est transférée dans ladite première poche, préalablement débarrassée de son milieu, l'ensemble des deux poches étant stockées dans des conditions préservant la viabilité cellulaire en vue de la co-culture entre les leucocytes pendant une période aboutissant à la production d'un surnageant conditionné.

8. Préparation pharmaceutique selon la revendication 7, **caractérisée en ce que** le surnageant résultant de ladite co-culture est collecté dans une nouvelle poche, filtré et conservé à très basse température.

9. Préparation pharmaceutique telle que définie dans l'une quelconque des revendications 1 à 8, pour son utilisation dans le traitement de cancers, seul ou en complément d'autres thérapies anti-cancéreuses.

10. Préparation pharmaceutique selon la revendication 9, **caractérisée en ce que** lesdits cancers sont notamment ceux représentatifs des lymphomes, leucémies, sarcomes et carcinomes, et plus précisément des lymphomes à cellules T, des lymphomes à cellules B, des mélanomes et des carcinomes du colon.

11. Préparation pharmaceutique pour son utilisation selon l'une des revendications 9 ou 10, **caractérisée en ce qu'**elle est le résultat d'un mélange de surnageants obtenus selon l'une quelconque des revendications 1 à 8 à partir de plusieurs sources leucocytaires.

## Patentansprüche

1. Pharmazeutisches Präparat zur Verwendung zur Kontrolle einer Tumorprogression oder zur Wiederherstellung einer Antitumorimmunität, umfassend einen Überstand, der aus einer Kokultur zwischen Makrophagen und bestrahlten Leukozyten erhalten wird.

2. Pharmazeutisches Präparat nach Anspruch 1, **dadurch gekennzeichnet, dass** die Makrophagen Leukozyten sind.

3. Pharmazeutisches Präparat nach den Ansprüchen 1 und 2, **dadurch gekennzeichnet, dass** die Leukozyten aus dem Buffy-Coat isoliert werden, der von Vollblut stammt.

4. Pharmazeutisches Präparat nach Anspruch 3, **dadurch gekennzeichnet, dass** ein Teil der Leukozyten gewaschen, in einem definierten Kulturmedium resuspendiert, dann in einen ersten Beutel, der einen Differenzierungsfaktor aufnimmt, und in Bedingungen, die die Zelllebensfähigkeit für mehrere Tage, vorzugsweise 3 aufrechterhalten, aufbewahrt, bevor erneut frisches definiertes Kulturmedium aufgenommen wird, und dann erneut für mehrere Tage, vorzugsweise 4 im Istzustand belassen wird, was zur Produktion von Makrophagen führt.

5. Pharmazeutisches Präparat nach Anspruch 3, **dadurch gekennzeichnet, dass** ein anderer Teil der Leukozyten in einen zweiten Beutel gegeben wird, der bei einer niedrigen Temperatur, vorzugsweise von weniger als +10 °C und noch mehr bevorzugt von weniger als +5 °C, aufbewahrt wird.

6. Pharmazeutisches Präparat nach Anspruch 5, **dadurch gekennzeichnet, dass** der zweite Beutel, nachdem er von seinem bestrahlten Medium befreit wurde, von seinem Medium befreit und dann wieder in einem Sekretionsmedium in Bedingungen, die die Zelllebensfähigkeit aufrechterhalten, aufgenommen wird.

7. Pharmazeutisches Präparat nach Anspruch 6, **dadurch gekennzeichnet, dass** der zweite Beutel in den ersten Beutel, der zuvor von seinem Medium befreit wurde, überführt wird, wobei die Einheit der zwei Beutel in Bedingungen, die die Zelllebensfähigkeit aufrechterhalten, zwecks der Kokultur zwischen den Leukozyten für einen Zeitraum gelagert wird, der zur Produktion eines konditionierten Überstands führt.

8. Pharmazeutisches Präparat nach Anspruch 7, **dadurch gekennzeichnet, dass** der aus der Kokultur resultierende Überstand in einem neuen Beutel gesammelt, filtriert und bei einer sehr niedrigen Temperatur aufbewahrt wird.

9. Pharmazeutisches Präparat, wie in einem der Ansprüche 1 bis 8 definiert, zu dessen Verwendung bei der Behandlung von Krebserkrankungen, allein oder ergänzend zu anderen Antikrebstherapien.

10. Pharmazeutisches Präparat nach Anspruch 9, **dadurch gekennzeichnet, dass** die Krebserkrankungen insbesondere diejenigen sind, die für Lymphome, Leukämien, Sarkome und Karzinome und genauer gesagt T-Zell-Lymphome, B-Zell-Lymphome, Melanome und Kolonkarzinome repräsentativ sind.

11. Pharmazeutisches Präparat zu dessen Verwendung nach einem der Ansprüche 9 oder 10, **dadurch gekennzeichnet, dass** es das Ergebnis eines Gemischs von Überständen ist, die nach einem der Ansprüche 1 bis 8 aus mehreren Leukozytenquellen erhalten werden.

## Claims

1. Pharmaceutical preparation to be used for controlling tumour progression or for restoring antitumour immunity, comprising a supernatant obtained from a coculture between macrophages and irradiated leucocytes.

2. Pharmaceutical preparation according to claim 1, **characterised in that** said macrophages are leucocytes.

3. Pharmaceutical preparation according to claims 1 and 2, **characterised in that** said leucocytes are isolated from the buffy coat resulting from whole blood.

4. Pharmaceutical preparation according to claim 3, **characterised in that** a portion of said leucocytes is washed, resuspended in a defined culture medium, then placed in a first pocket receiving a differentiation factor and preserved in conditions which preserve cellular viability for several days, preferably 3, prior to again receiving fresh defined culture medium, then left as it is again for several days, preferably 4, resulting in the production of macrophages.

5. Pharmaceutical preparation according to claim 3, **characterised in that** another portion of said leucocytes is placed in a second pocket kept at a low temperature, preferably below +10°C, and more preferably below +5°C.

6. Pharmaceutical preparation according to claim 5, **characterised in that** said second pocket, after having been freed from its irradiated medium, is freed from its medium, then put in a secretion medium in conditions that preserve cellular viability.

7. Pharmaceutical preparation according to claim 6, **characterised in that** said second pocket is transferred into the said first pocket, previously freed from its medium, the set of two pockets being stored in conditions that preserve cellular viability for forming the co-culture between the leucocytes for a period leading to the production of a conditioned supernatant.

8. Pharmaceutical preparation according to claim 7, **characterised in that** the resulting supernatant of said co-culture is collected in a new pocket, filtered and kept at a very low temperature.

9. Pharmaceutical preparation as defined in any of claims 1 to 8, to be used in the treatment of cancers, either alone or in addition to other anti-cancer therapies.

10. Pharmaceutical preparation according to claim 9, **characterised in that** said cancers are in particular representatives of lymphomas, leukaemias, sarcomas and carcinomas, and more precisely T-cell lymphomas, B-cell lymphomas, melanomas and colon carcinomas.

11. Pharmaceutical preparation for use according to any of claims 9 or 10, **characterised in that** it is the result of a mixture of supernatants obtained according to any of claims 1 to 8 from several leucocyte sources.
